(19) Europäisches Patentamt
European Patent Office
Office européen des brevets

(11) Veröffentlichungsnummer : **0 040 744**
**B1**

(12) **EUROPÄISCHE PATENTSCHRIFT**

(45) Veröffentlichungstag der Patentschrift : **08.02.84**

(21) Anmeldenummer : **81103583.1**

(22) Anmeldetag : **11.05.81**

(51) Int. Cl.³ : **C 07 D295/12**, C 07 D209/44, C 07 D265/30, C 07 C131/00, A 01 N 35/10, A 01 N 43/38

# (54) Aminopropiophenon-oxime, Verfahren zu ihrer Herstellung und ihre Verwendung als Fungizide.

(30) Priorität : **22.05.80 DE 3019497**

(43) Veröffentlichungstag der Anmeldung : **02.12.81 Patentblatt 81/48**

(45) Bekanntmachung des Hinweises auf die Patenterteilung : **08.02.84 Patentblatt 84/06**

(84) Benannte Vertragsstaaten : **AT BE CH DE FR GB IT LI NL SE**

(56) Entgegenhaltungen :
**EP-A- 0 004 000**
**DD-A- 124 521**
**DE-A- 2 056 785**
**DE-A- 2 823 742**
**DE-A- 2 842 091**
**GB-A- 1 213 963**
**GB-A- 1 452 868**
**Chemical Abstracts Band 88, Nr. 3, 16. Januar 1978, Columbus, Ohio, USA, Y. YOKOYAMA et al. "4'-Substituted-2-methyl-3-piperidinopropiophenones" Seite 612, Abstract Nr. 22646j**
**Die Akte enthält technische Angaben, die nach dem Eingang der Anmeldung eingereicht wurden und die nicht in dieser Patentschrift enthalten sind.**

(73) Patentinhaber : **BAYER AG**
**Zentralbereich Patente, Marken und Lizenzen**
**D-5090 Leverkusen 1, Bayerwerk (DE)**

(72) Erfinder : **Knops, Hans-Joachim, Dr.**
**Claudiusweg 3**
**D-5600 Wuppertal-1 (DE)**
Erfinder : **Krämer, Wolfgang, Dr.**
**Am Eckbusch 39/45**
**D-5600 Wuppertal-1 (DE)**
Erfinder : **Frohberger, Paul-Ernst, Dr.**
**Willi-Baumeister-Strasse 5**
**D-5090 Leverkusen-1 (DE)**

**EP 0 040 744 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Aminopropiophenon-oxime, Verfahren zu ihrer Herstellung und ihre Verwendung als Fungizide

Die Erfindung betrifft neue Aminopropiophenon-oxime, mehrere Verfahren zu ihrer Herstellung und ihre Verwendung als Fungizide.

Es ist bereits bekannt geworden, daß Triazolyl-ethanol-Derivate, wie z. B. 1-(4-Chlorphenyl)-2-(1,2,4-triazol-1-yl)-1-ethanol, im allgemeinen gute fungizide Eigenschaften aufweisen (vergleiche DE-OS 24 31 407).

Die Wirkung ist jedoch, insbesondere bei niedrigen Aufwandmengen und -konzentrationen, in einigen Anwendungsbereichen nicht immer voll befriedigend.

Weiterhin sind Aminopropiophenon-oxime, wie z. B. das 3-Diethylamino-propiophenon-oxim bekannt (vgl. DE-OS 20 56 785). Diese Aminopropiophenon-oxime werden als Ausgangsverbindungen zur Herstellung pharmakologisch wirksamer Verbindungen, wie z. B. Carbamoyl-oximen, verwendet. Über eine Wirkung auf dem Pflanzenschutzsektor ist nichts bekannt (vgl. DE-OS 20 56 785).

Es wurden nun neue Aminopropiophenon-oxime der allgemeinen Formel I

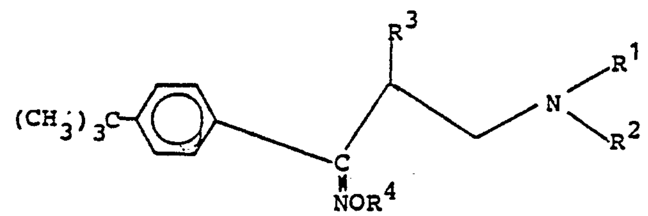

(I)

in welcher

$R^1$ und $R^2$ für Alkyl mit 1 bis 4 Kohlenstoffatomen stehen ; oder gemeinsam mit dem Stickstoffatom, an das sie gebunden sind, für die gegebenenfalls durch Alkyl mit 1 bis 4 Kohlenstoffatomen oder durch einen gegebenenfalls durch Alkyl mit 1 bis 4 Kohlenstoffatomen und Halogen substituierten ankondensierten aromatischen oder alicyclischen Ring mit 5 bis 7 Kohlenstoffatomen substituierten Heterocyclen

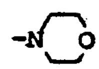 , 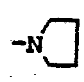 , 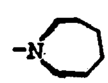 und 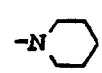

stehen,

$R^3$ für Wasserstoff oder Alkyl mit 1 bis 4 Kohlenstoffatomen steht, und

$R^4$ für Wasserstoff, Alkyl mit 1 bis 4 Kohlenstoffatomen, Alkenyl und Alkinyl mit jeweils 2 bis 4 Kohlenstoffatomen oder für gegebenenfalls durch Halogen, Alkyl mit 1 bis 4 Kohlenstoffatomen und Halogenalkyl mit 1 oder 2 Kohlenstoffatomen und 2 bis 5 Halogenatomen, Alkoxy und Alkylthio mit jeweils 1 oder 2 Kohlenstoffatomen, Cyano und Nitro substituiertes Aralkyl mit 6 bis 10 Kohlenstoffatomen im Arylteil und 1 oder 2 Kohlenstoffatomen im Alkylteil steht,

und deren physiologisch verträglichen Säureadditions-Salze gefunden.

Die Verbindungen der allgemeinen Formel I können in der syn- und anti-Form vorliegen ; vorwiegend fallen sie als Gemische beider Formen an.

Weiterhin wurde gefunden, daß man die Aminopropiophenon-oxime der allgemeinen Formel I erhält, wenn man

a) Aminopropiophenone der allgemeinen Formel II

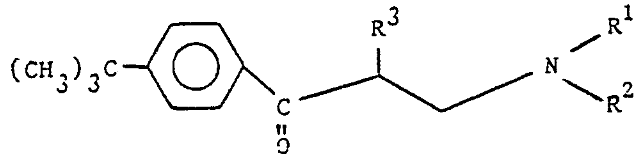

(II)

in welcher

$R^1$, $R^2$ und $R^3$ die oben angegebene Bedeutung haben,

mit Salzen von Hydroxylamin-(Derivaten) der allgemeinen Formel III

$$H_2N—O—R^4 \qquad (III)$$

in welcher

$R^4$ die oben angegebene Bedeutung hat,
in Gegenwart eines Verdünnungsmittels und in Gegenwart eines Säurebindemittels umsetzt, und/oder

b) gegebenenfalls Alkalisalze von nach Verfahren (a) erhaltenen Oxim-Derivaten der allgemeinen Formel IV

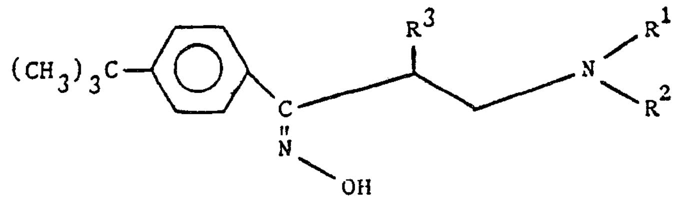

(IV)

in welcher

$R^1$, $R^2$ und $R^3$ die oben angegebene Bedeutung haben,
mit Halogeniden der allgemeinen Formel V

$$Z{-}R^5 \qquad (V)$$

in welcher

$R^5$ für Alkyl mit 1 bis 4 Kohlenstoffatomen, Alkenyl und Alkinyl mit jeweils 2 bis 4 Kohlenstoffatomen oder für gegebenenfalls durch Halogen, Alkyl mit 1 bis 4 Kohlenstoffatomen, Halogenalkyl mit 1 oder 2 Kohlenstoffatomen und 2 bis 5 Halogenatomen, Alkoxy und Alkylthio mit jeweils 1 oder 2 Kohlenstoffatomen, Cyano und Nitro substituiertes Aralkyl mit 6 bis 10 Kohlenstoffatomen im Arylteil und 1 oder 2 Kohlenstoffatomen im Alkylteil steht und

Z für Chlor oder Brom steht,
in Gegenwart eines organischen Verdünnungsmittels oder in Gegenwart eines organisch-anorganischen Zweiphasensystems in Gegenwart eines Phasentransferkatalysators umsetzt, wobei die Alkalisalze der Oxime der allgemeinen Formel IV in situ erzeugt werden.

An die Verbindungen der allgemeinen Formel I kann gegebenenfalls noch eine physiologisch verträgliche Säure angelagert werden.

Die neuen Aminopropiophenon-oxime der allgemeinen Formel I weisen starke fungizide Eigenschaften auf. Dabei zeigen überraschenderweise die erfindungsgemäßen Verbindungen eine höhere Wirkung als das aus dem Stand der Technik bekannte 1-(4-Chlorphenyl)-2-(1,2,4-triazol-1-yl)-1-ethanol, welches eine Verbindung gleicher Wirkungsrichtung ist. Die erfindungsgemäßen Stoffe stellen somit eine Bereicherung der Technik dar.

Die erfindungsgemäßen Aminopropiophenon-oxime sind durch die allgemeine Formel I definiert. In dieser Formel sind $R^1$ und $R^2$ gleich oder verschieden und stehen für geradkettiges oder verzweigtes Alkyl mit 1 bis 4 Kohlenstoffatomen. $R^1$ und $R^2$ stehen außerdem gemeinsam mit dem Stickstoffatom, an das sie gebunden sind, für die gegebenenfalls substituierten Heterocyclen

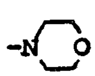 , 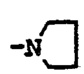 , 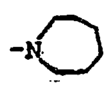 sowie 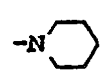 wobei

als Substituenten infrage kommen : Alkyl mit 1 bis 4 Kohlenstoffatomen sowie ein gegebenenfalls durch Alkyl mit 1 bis 4 Kohlenstoffatomen und Halogen substituierter ankondensierter aromatischer oder alicyclischer Ring mit 5 bis 7 Kohlenstoffatomen.

$R^3$ steht für Wasserstoff oder Alkyl mit 1 bis 4 Kohlenstoffatomen und

$R^4$ für Wasserstoff, geradkettiges oder verzweigtes Alkyl mit 1 bis 4 Kohlenstoffatomen, Alkenyl und Alkinyl mit jeweils 2 bis 4 Kohlenstoffatomen sowie für gegebenenfalls substituiertes Aralkyl mit 6 bis 10 Kohlenstoffatomen im Arylteil und 1 bis 2 Kohlenstoffatomen im Alkylteil, wie insbesondere Benzyl, steht, wobei als Substituenten infrage kommen: Halogen, Alkyl mit 1 bis 4 Kohlenstoffatomen, Halogenalkyl mit 1 bis 2 Kohlenstoff- und 2 bis 5 Halogenatomen, wobei als Halogene vorzugsweise Fluor und Chlor genannt seien, Alkoxy und Alkylthio mit jeweils 1 oder 2 Kohlenstoffatomen, sowie Cyano und Nitro.

Besonders bevorzugt sind diejenigen Aminopropiophenon-oxime der allgemeinen Formel I, in denen $R^1$ und $R^2$ für Alkyl mit 1 bis 4 Kohlenstoffatomen stehen, gemeinsam mit dem Stickstoffatom, an das sie gebunden sind, für die gegebenenfalls substituierten Heterocyclen

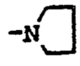 , 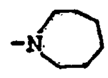 , 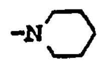 und 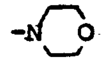

stehen, wobei als Substituenten infrage kommen : Methyl, Ethyl sowie ein ankondensierter Benzol- oder Cyclohexylring ; $R^3$ für Wasserstoff oder Methyl steht und $R^4$ für Wasserstoff, Methyl, Ethyl, Isopropyl, n-Propyl, n-Butyl, tert.-Butyl, Vinyl, Allyl, Propargyl sowie für gegebenenfalls durch Chlor, Methyl oder Trifluormethyl substituiertes Benzyl steht.

Im einzelnen seien außer den bei den Herstellungsbeispielen genannten Verbindungen die folgenden Verbindungen der allgemeinen Formel I genannt :

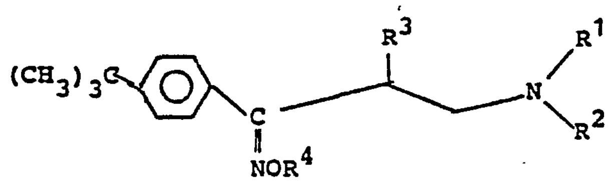

(I)

| $R^4$ | $R^3$ | 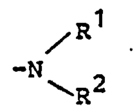 |
|---|---|---|
| $C_3H_7$-i | $CH_3$ | 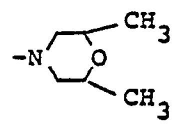 |
| $C_3H_7$-n | $CH_3$ | 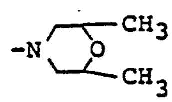 |
| $-CH_2-CH=CH_2$ | $CH_3$ | 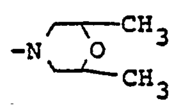 |
| $-CH_2-C\equiv CH$ | $CH_3$ | 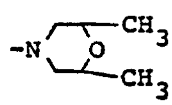 |
| $-CH_2-C\equiv CH$ | $CH_3$ | 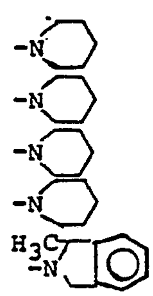 |
| $-CH_2-CH=CH_2$ | $CH_3$ | |
| $C_3H_7$-n | $CH_3$ | |
| $C_3H_7$-i | $CH_3$ | |
| H | $CH_3$ | |
| H | $CH_3$ | $-N(C_2H_5)_2$ |
| H | $CH_3$ | $-N(C_3H_7\text{-i})_2$ |
| H | $CH_3$ | $-N(C_3H_7\text{-n})_2$ |
| H | $CH_3$ | $-N(C_4H_9\text{-n})_2$ |
| H | $CH_3$ | $-N(C_4H_9\text{-i})_2$ |

Verwendet man p-tert.-Butyl-2-methyl-3-morpholin-4-yl-propiophenon und Hydroxylamin-hydrochlorid als Ausgangsstoffe, so kann der Reaktionsablauf durch das folgende Formelschema wiedergegeben werden (Verfahren a) :

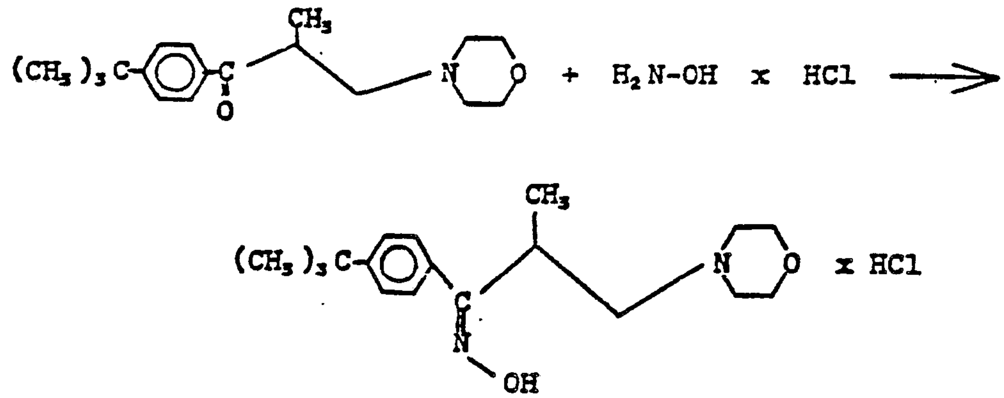

Verwendet man das Natriumsalz von p-tert.-Butyl-2-methyl-3-morpholin-4-yl-propiophenon-oxim und Benzylchlorid als Ausgangsstoffe, so kann der Reaktionsablauf durch das folgende Formelschema wiedergegeben werden (Verfahren b):

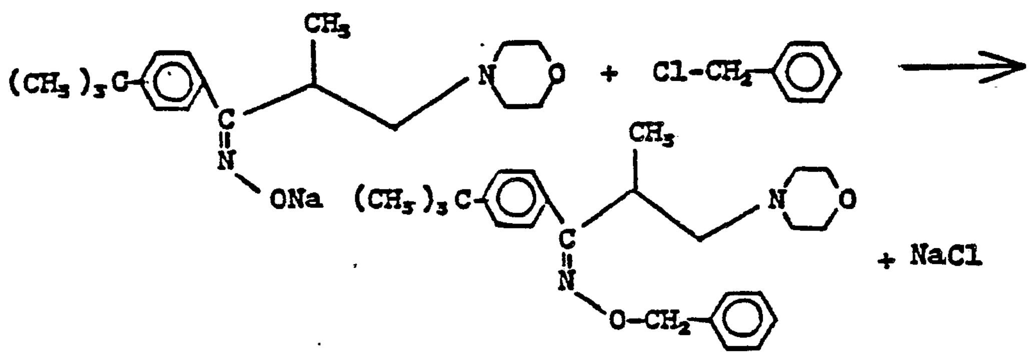

Die für das erfindungsgemäße Verfahren (a) als Ausgangsstoffe zu verwendenden Ketone der allgemeinen Formel II sind teilweise bekannt und können nach bekannten Verfahren aus Acetophenonen mit Paraformaldehyd und mit Aminen in Gegenwart eines Verdünnungsmittels erhalten werden.

Die für das erfindungsgemäße Verfahren (b) als Ausgangsstoffe zu verwendenden Oxime der allgemeinen Formel IV sind erfindungsgemäße Verbindungen.

Die außerdem für das erfindungsgemäße Verfahren (a) als Ausgangsstoffe (in Form ihrer Salze) zu verwendenden Hydroxylamin-(Derivate) sind durch die allgemeine Formel (III) definiert. Die Verbindungen der allgemeinen Formel (III) werden vorzugsweise in Form ihrer Hydrohalogenide, insbesondere als Hydrochlorid, eingesetzt.

Die außerdem für das erfindungsgemäße Verfahren (b) als Ausgangsstoffe zu verwendenden halogenide sind durch die allgemeine Formel V definiert. In dieser Formel steht $R^5$ für geradkettiges oder verzweigtes Alkyl mit 1 bis 4 Kohlenstoffatomen, Alkenyl oder Alkinyl mit jeweils 2 bis 4 Kohlenstoffatomen sowie für gegebenenfalls substituiertes Aralkyl mit 6 bis 10 Kohlenstoffatomen im Arylteil und 1 bis 2 Kohlenstoffatomen im Alkylteil, wie insbesondere Benzyl, wobei als Substituenten infrage kommen: Halogen, Alkyl mit 1 bis 4 Kohlenstoffatomen, Halogenalkyl mit 1 bis 2 Kohlenstoff- und 2 bis 5 Halogenatomen, wobei als Halogene vorzugsweise Fluor und Chlor genannt seien, Alkoxy und Alkylthio mit jeweils 1 oder 2 Kohlenstoffatomen, sowie Cyano und Nitro.

Als Verdünnungsmittel kommen für das erfindungsgemäße Verfahren (a) vorzugsweise protische Lösungsmittel infrage. Hierzu gehören insbesondere Alkohole bzw. wäßrige Alkohole, wie beispielsweise Ethanol.

Die Reaktionstemperaturen können bei der Durchführung des erfindungsgemäßen Verfahrens (a) in einem größeren Bereich variiert werden. Im allgemeinen arbeitet man zwischen 20 und 120 °C, vorzugsweise zwischen 40 und 100 °C.

Bei der Durchführung des erfindungsgemäßen Verfahrens (a) setzt man vorzugsweise auf 1 Mol Keton der allgemeinen Formel II 1 bis 2 Mol der Verbindung der allgemeinen Formel III ein. Da die Verbindungen der allgemeinen Formel III in Form ihrer Salze, vorzugsweise als Hydrochloride eingesetzt werden, können die Endprodukte auch direkt als Salze isoliert werden; es kann aber auch in üblicher Weise die entsprechende Base freigesetzt werden. Es kann auch in Gegenwart eines Säurebindemittels, wie vorzugsweise Alkalicarbonate und -acetate, gearbeitet werden. Die Aufarbeitung und Isolierung erfolgt jeweils nach üblichen Methoden.

Als Verdünnungsmittel kommen für das erfindungsgemäße Verfahren (b) alle inerten organischen Lösungsmittel infrage. Hierzu gehören vorzugsweise Ether, wie Diethylether und Dioxan ; aromatische Kohlenwasserstoffe, wie Toluol und Benzol ; in einzelnen Fällen auch chlorierte Kohlenwasserstoffe, wie Chloroform, Methylenchlorid und Tetrachlorkohlenstoff.

Die Reaktionstemperaturen können bei der Durchführung des erfindungsgemäßen Verfahrens (b) in einem größeren Bereich variiert werden. Im allgemeinen arbeitet man zwischen 20 und 150 °C, vorzugsweise bei Raumtemperatur. In einzelnen Fällen ist es vorteilhaft, bei der Siedetemperatur des Lösungsmittels, beispielsweise zwischen 60 und 100 °C, zu arbeiten.

Bei der Durchführung des erfindungsgemäßen Verfahrens (b) setzt man auf 1 Mol Alkalisalz eines Oxims der allgemeinen Formel IV vorzugsweise 1 bis 3 Mol Halogenid der allgemeinen Formel V ein.

Die Aufarbeitung und Isolierung erfolgt nach üblichen Methoden.

In einer bevorzugten Ausführungsform des Verfahrens (b) wird zweckmäßigerweise so verfahren, daß man von einem Oxim der allgemeinen Formel IV ausgeht, letzteres in einem geeigneten inerten organischen Lösungsmittel mittels Alkalimetallhydrid oder -amid in das Salz überführt, und letzteres ohne Isolierung sofort mit einem Halogenid der allgemeinen Formel V umsetzt, wobei unter Austritt von Alkalihalogenid die erfindungsgemäßen Verbindungen in einem Arbeitsgang erhalten werden.

Nach einer weiteren bevorzugten Ausführungsform des Verfahrens (b) werden zweckmäßigerweise die Herstellung der Salze der Oxime der allgemeinen Formel IV sowie die erfindungsgemäße Umsetzung in einem Zweiphasensystem, wie beispielsweise wässrige Natron- oder Kalilauge/Toluol oder Methylenchlorid, unter Zusatz von 0,1-1 Mol eines Phasen-Transfer-Katalysators, wie beispielsweise einer Ammonium- oder Phosphoniumverbindung, durchgeführt.

In einer weiteren bevorzugten Ausführungsform des Verfahrens (b) wird so verfahren, daß man das Oxim der allgemeinen Formel IV in Gegenwart von Alkalicarbonaten, wie insbesondere Kaliumcarbonat, in Gegenwart eines organischen Lösungsmittels, wie insbesondere Dimethylformamid, umsetzt.

Zur Herstellung von physiologisch verträglichen Säureadditions-Salzen der Verbindungen der allgemeinen Formel I kommen vorzugsweise folgende Säuren infrage : Die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionnelle Carbonsäuren und Hydroxycarbonsäuren, wie z. B. Essigsäure, Maleinsäure, Bernsteinsäure, Fumarsäure, Weinsäure, Citronensäure, Salicylsäure, Sorbinsäure, Milchsäure, sowie Sulfonsäuren, wie z. B. p-Toluolsulfonsäure und 1,5-Naphthalindisulfonsäure.

Die Säureadditionssalze der Verbindungen der allgemeinen Formel I können in einfacher Weise nach üblichen Salzbildungsmethoden, z. B. durch Lösen einer Verbindung der allgemeinen Formel I in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zugomycetes, Ascomycetes, Basidiomycetes und Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen, so zur Bekämpfung von Erysiphe-Arten, wie z. B. gegen den Erreger des Gersten- bzw. des Getreidemehltaus (Erysiphe graminis) ; sowie zur Bekämpfung solcher Pilze, die Schorf- und Rostkrankheiten hervorrufen, so zur Bekämpfung von Venturia-Arten, wie z. B. gegen den Erreger des Apfelschorfs (Fusicladium dendriticum) und von Uromyces-Arten, wie z. B. gegen den Erreger des Bohnenrostes (Uromyces phaseoli).

Außerdem zeigen die erfindungsgemäßen Wirkstoffe eine gute fungizide in-vitro-Wirkung gegen Fusarium nivale.

In bestimmten Aufwandmengen zeigen die erfindungsgemäßen Stoffe auch eine wachstumsregulierende Wirkung.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organiche Lösungsmittel als Hilfslösungsmittel

verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glykol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit verflüssigten gas förmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid ; als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo-, und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 %, am Wirkungsort erforderlich.

## Beispiel 1

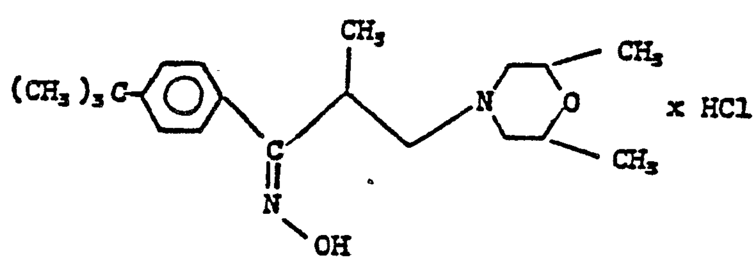

(Verfahren a)

9,5 g (0,03 Mol) p-tert.-Butyl-2-methyl-3-(2,6-dimethyl-morpholin-4-yl)-propiophenon werden mit 3,6 g (0,05 Mol) Hydroxylamin-hydrochlorid in 80 ml Ethanol 20 Stunden unter Rückfluß erhitzt. Nach dem Abkühlen auf 5 °C wird filtriert. Das Filtrat wird eingeengt, mit verdünnter Natronlauge aufgeschlämmt und mit Chloroform extrahiert. Die organische Phase wird mit etherischer Salzsäure versetzt und das ausgefallene Reaktionsprodukt abgesaugt. Man erhält so 10,6 g (96 % der Theorie) p-tert.-Butyl-2-methyl-3-(2,6-dimethyl-morpholin-4-yl)-propiophenon-oxim-hydrochlorid vom Schmelzpunkt 214-16 °C.

Beispiel 2

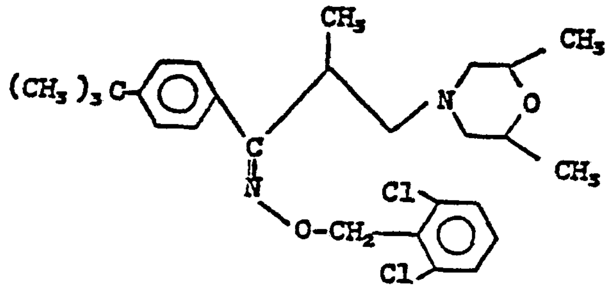

x HCl

(Verfahren a)

9,5 g (0,03 Mol) p-tert.-Butyl-2-methyl-3-(2,6-dimethyl-morpholin-4-yl)-propiophenon, 6,2 g (0,03 Mol) 2,6-Dichlor-benzyl-hydroxylamin-hydrochlorid und 2,2 g Natriumacetat werden in 60 ml Ethanol 55 Stunden unter Rückfluß gerührt. Nach dem Abkühlen wird abgesaugt, das Filtrat eingeengt und in 50 ml Methylenchlorid aufgenommen. Man wäscht mit Wasser, Natriumhydrogencarbonatlösung und wiederum mit Wasser, trocknet über Natriumsulfat und engt ein. Der ölige Rückstand wird in Petrolether gelöst und filtriert. Das Filtrat wird mit Aktivkohle behandelt und erneut eingeengt. Der Rückstand wird in Ether aufgenommen, mit etherischer Salzsäure versetzt und eingeengt. Man erhält so 5 g (35 % der Theorie) p-tert.-Butyl-2-methyl-3-(2,6-dimethylmorpholin-4-yl)-propiophenon-oxim-0-2,6-dichlorbenzyl-ether-hydrochlorid vom Schmelzpunkt 158-67 °C.

In entsprechender Weise und gemäß den angegebenen Verfahren werden die nachstehenden Beispiele der allgemeinen Formel I

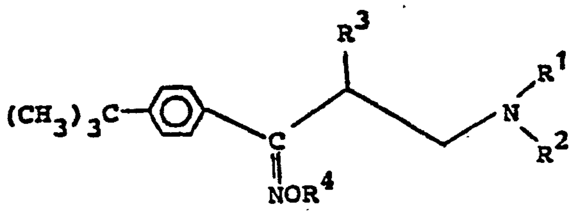

(I)

erhalten :

| Bsp. Nr. | $R^4$ | $R^3$ | 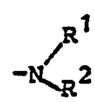 | Schmelzpunkt (°C) bzw. Siedepunkt (°C /mmHg-Säule) |
|---|---|---|---|---|
| 3 | H | $CH_3$ | 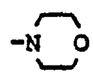 | 202-03 (Zers.) / (xHCl) |
| 4 | H | $CH_3$ | 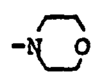 | Oel |
| 5 | H | $CH_3$ | 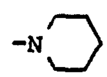 | 214-16 (Zers.) / (xHCl) |
| 6 | H | $CH_3$ | 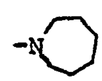 | 194-97 (xHCl) |
| 7 | 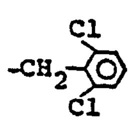 | $CH_3$ | 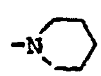 | Oel |
| 8 | H | $CH_3$ | 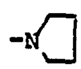 | 189-92 (xHCl) |

Tabelle (Fortsetzung)

| Bsp. Nr. | $R^4$ | $R^3$ | 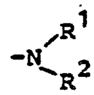 | Schmelzpunkt (°C) bzw. Siedepunkt (°C/ mmHg-S.) |
|---|---|---|---|---|
| 9 | H | $CH_3$ | $-N(CH_3)_2$ | 170-72 (xHCl) |
| 10 | $-CH_2-CH=CH_2$ | $CH_3$ | 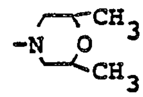 | 144-52 (xHCl) |
| 11 | H | $CH_3$ | $-N(C_3H_7)_2$ | 129 (xHCl) |
| 12 | H | $CH_3$ | $-N(C_2H_5)_2$ | 125 (xHCl) |
| 13 | $-CH_2-C\equiv CH$ | $CH_3$ | 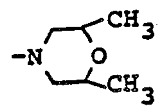 | Oel |
| 14 | $-CH(CH_3)_2$ | $CH_3$ | 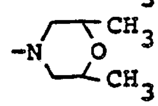 | Oel |
| 15 | $-CH_2-CH_2-CH_3$ | $CH_3$ | 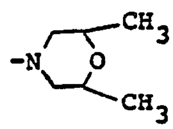 | Oel |
| 16 | H | $CH_3$ | 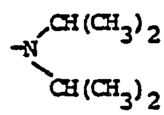 | Oel |
| 17 | H | $CH_3$ | 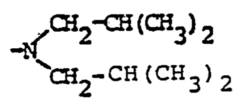 | Oel |
| 18 | H | $CH_3$ | 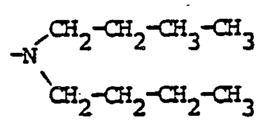 | Oel |

## Verwendungsbeispiele

In dem nachfolgenden Beispiel wird die nachstehend angegebene Verbindung als Vergleichssubstanz eingesetzt:

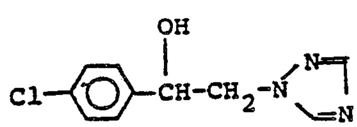

(A)

### Beispiel A

Erysiphe-Test (Gerste)/protektiv/

Lösungsmittel: 100 Gewichtsteile Dimethylformamid
Emulgator: 0,25 Gewichtsteile Alkyl-aryl-polyglykol-ether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Ueberlegenheit in der Wirksamkeit gegenüber dem Stand der Technik zeigen bei diesem Test z. B. die Verbindungen gemäß folgender Herstellungsbeispiele : 1, 2, 3 und 5.

**Ansprüche**

1. Aminopropiophenon-oxime der allgemeinen Formel I

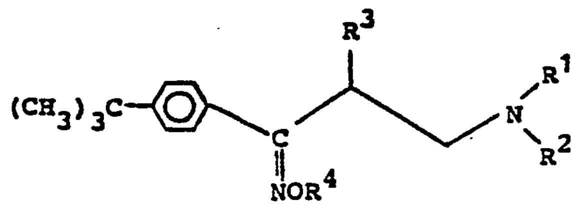

(I)

in welcher

$R^1$ und $R^2$ für Alkyl mit 1 bis 4 Kohlenstoffatomen stehen ; oder gemeinsam mit dem Stickstoffatom, an das sie gebunden sind, für die gegebenenfalls durch Alkyl mit 1 bis 4 Kohlenstoffatomen oder durch einen gegebenenfalls durch Alkyl mit 1 bis 4 Kohlenstoffatomen und Halogen substituierten ankondensierten aromatischen oder alicyclischen Ring mit 5 bis 7 Kohlenstoffatomen substituierten Heterocyclen

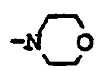 , 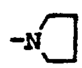 , 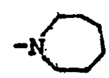 sowie 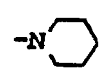

stehen,

$R^3$ für Wasserstoff oder Alkyl mit 1 bis 4 Kohlenstoffatomen steht und

$R^4$ für Wasserstoff, Alkyl mit 1 bis 4 Kohlenstoffatomen, Alkenyl und Alkinyl mit jeweils 2 bis 4 Kohlenstoffatomen oder für gegebenenfalls durch Halogen, Alkyl mit 1 bis 4 Kohlenstoffatomen, Halogenalkyl mit 1 oder 2 Kohlenstoffatomen und 2 bis 5 Halogenatomen, Alkoxy und Alkylthio mit jeweils 1 oder 2 Kohlenstoffatomen, Cyano und Nitro substituiertes Aralkyl mit 6 bis 10 Kohlenstoffatomen im Arylteil und 1 oder 2 Kohlenstoffatomen im Alkylteil steht,

und deren physiologisch verträgliche Säureadditions-Salze.

2. Verfahren zur Herstellung von Aminopropiophenon-oximen der allgemeinen Formel I in Anspruch 1, dadurch gekennzeichnet, daß man

a) Aminopropionphenone der allgemeinen Formel II

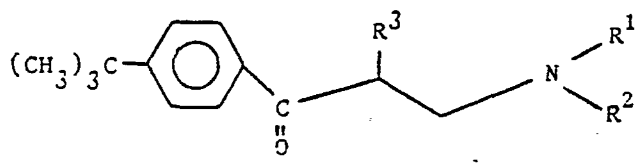

(II)

in wlecher

$R^1$, $R^2$ und $R^3$ die in Anspruch 1 angegebene Bedeutung haben,

mit Salzen von Hydroxylamin-(Derivaten) der allgemeinen Formel III

$$H_2N—O—R^4 \qquad (III)$$

in welcher

$R^4$ die oben angegebene Bedeutung hat,

in Gegenwart eines Verdünnungsmittels und in Gegenwart eines Säurebindemittels umsetzt, und/oder

b) gegebenenfalls Alkalisalze von nach Verfahren (a) erhaltenen Oxim-Derivaten der allgemeinen Formel IV

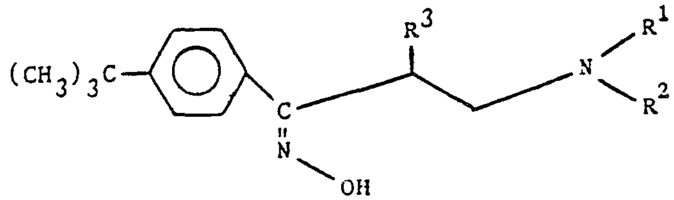

(IV)

in welcher

$R^1$, $R^2$ und $R^3$ die in Anspruch 1 angegebene Bedeutung haben,

mit Halogeniden der allgemeinen Formel V

$$Z—R^5 \qquad (V)$$

in welcher

$R^5$ für Alkyl mit 1 bis 4 Kohlenstoffatomen, Alkenyl und Alkinyl mit jeweils 2 bis 4 Kohlenstoffatomen oder für gegebenenfalls durch Halogen, Alkyl mit 1 bis 4 Kohlenstoffatomen, Halogenalkyl mit 1 oder 2 Kohlenstoffatomen und 2 bis 5 Halogenatomen, Alkoxy und Alkylthio mit jeweils 1 oder 2 Kohlenstoffatomen, Cyano und Nitro substituiertes Aralkyl mit 6 bis 10 Kohlenstoffatomen im Arylteil und 1 oder 2 Kohlenstoffatomen im Alkylteil steht und

Z für Chlor oder Brom steht,

in Gegenwart eines organischen Verdünnungsmittels oder in Gegenwart eines organisch-anorganischen Zweiphasensystem in Gegenwart eines Phasentransferkatalysators umsetzt, und noch gegebenenfalls an die so erhaltenen Verbindungen der allgemeinen Formel I eine physiologisch verträgliche Säure anlagert.

3. Fungizide Mittel, gekennzeichnet durch einen Gehalt an mindestens einem Aminopropiophenon-oxim der allgemeinen Formel I.

4. Verfahren zur Bekämpfung von Pilzen auf pflanzlichen und technischen Materialien dadurch gekennzeichnet, daß man Aminopropiophenon-oxime der allgemeinen Formel I auf Pilze oder ihren Lebensraum einwirken läßt.

5. Verwendung von Aminopropiophenon-oximen der allgemeinen Formel I zur Bekämpfung von Pilzen auf pflanzlichen und technischen Materialien.

6. Verfahren zur Herstellung von fungiziden Mitteln, dadurch gekennzeichnet, daß man Aminopropiophenon-oxime der allgemeinen Formel I mit Streckmitteln und/oder oberflächenaktiven Mitteln vermischt.

**Claims**

1. Aminopropiophenone oximes of the general formula I

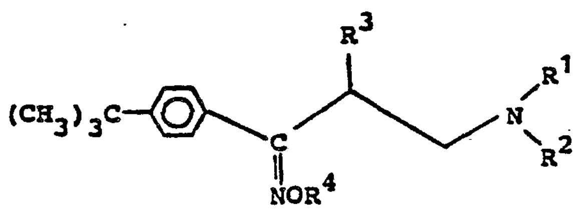

(I)

in which

$R^1$ and $R^2$ represent alkyl with 1 to 4 carbon atoms ; or together with the nitrogen atom to which they are bonded, represent the heterocyclic radicals

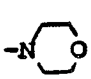 , 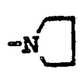 , 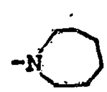 or 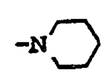

which are optionally substituted by alkyl with 1 to 4 carbon atoms or by a fused-on aromatic or alicyclic ring which has 5 to 7 carbon atoms and is optionally substituted by alkyl with 1 to 4 carbon atoms or halogen,

$R^3$ represents hydrogen or alkyl with 1 to 4 carbon atoms and

$R^4$ represents hydrogen, alkyl with 1 to 4 carbon atoms, alkenyl or alkinyl with in each case 2 to 4 carbon atoms or aralkyl which has 6 to 10 carbon atoms in the aryl part and 1 or 2 carbon atoms in the alkyl part and is optionally substituted by halogen, alkyl with 1 to 4 carbon atoms, halogeno-alkyl with 1 or 2 carbon atoms and 2 to 5 halogen atoms, alkoxy or alkylthio with in each case 1 or 2 carbon atoms, cyano or nitro,

and physiologically acceptable acid addition salts thereof.

2. Process for the preparation of aminopropiophenone oximes of the general formula I in Claim 1, characterised in that

a) aminopropiophenones of the general formula II

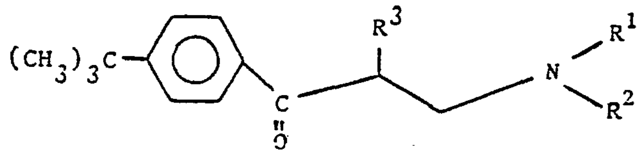

(II)

in which

$R^1$, $R^2$ and $R^3$ have the meaning given in Claim 1,

are reacted with salts of hydroxylamine (derivatives) of the general formula III

$$H_2N—O—R^4 \quad (III)$$

in which

$R^4$ has the meaning indicated above,

in the presence of a diluent and in the presence of an acid-binding agent, and/or

b) if appropriate, alkali metal salts of oxime derivatives, obtained by process a), of the general formula IV

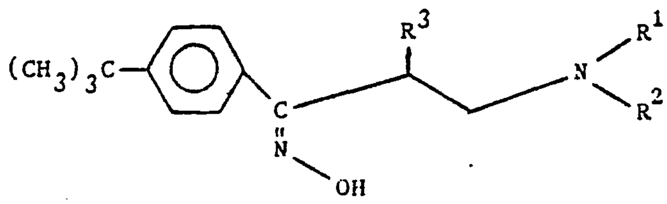

(IV)

in which

$R^1$, $R^2$ and $R^3$ have the meaning indicated in Claim 1,

are reacted with halides of the general formula V

$$Z—R^5 \quad (V)$$

in which

$R^5$ represents alkyl with 1 to 4 carbon atoms, alkenyl or alkinyl with in each case 2 to 4 carbon atoms or aralkyl which has 6 to 10 carbon atoms in the aryl part and 1 or 2 carbon atoms in the alkyl part and is optionally substituted by halogen, alkyl with 1 to 4 carbon atoms, halogenoalkyl with 1 or 2 carbon atoms and 2 to 5 halogen atoms, alkoxy or alkylthio with in each case 1 or 2 carbon atoms, cyano or nitro, and

Z represents chlorine or bromine,

in the presence of an organic diluent of in the presence of an organic/inorganic two-phase system in the presence of a phase transfer catalyst, and a physiologically acceptable acid is then optionally added on to the resulting compounds of the general formula I.

3. Fungicidal agents, characterised in that they contain at least one aminopropiophenone oxime of the general formula I.

4. Process for combating fungi on plant and industrial materials, characterised in that aminopropiophenone oximes of the general formula I are allowed to act on fungi or their environment.

5. Use of aminopropiophenone oximes of the general formula I for combating fungi on plant and industrial materials.

6. Process for the preparation of fungicidal agents, characterised in that aminopropiophenone oximes of the general formula I are mixed with extenders and/or surface-active agents.

**Revendications**

1. Aminopropiophénone-oximes de formule générale I

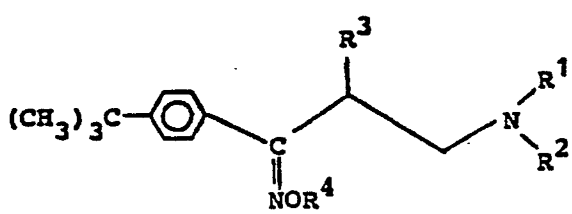

(I)

dans laquelle

$R^1$ et $R^2$ représentent chacun un groupe alkyle contenant 1 à 4 atomes de carbone ou, ensemble avec l'atome d'azote auquel ils sont reliés, ils représentent les noyaux hétérocycliques

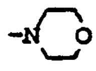 , 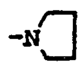 , 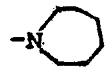 et 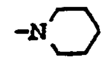

éventuellement substitués par un groupe alkyle contenant 1 à 4 atomes de carbone ou par un noyau alicyclique ou aromatique condensé en $C_5$-$C_7$ éventuellement substitué par un groupe alkyle contenant 1 à 4 atomes de carbone et par un atome d'halogène,

$R^3$ représente un atome d'hydrogène ou un groupe alkyle contenant 1 à 4 atomes de carbone, et

$R^4$ représente un atome d'hydrogène, un groupe alkyle contenant 1 à 4 atomes de carbone, un groupe alcényle et un groupe alcinyle contenant chacun 2 à 4 atomes de carbone ou un groupe aralkyle contenant 6 à 10 atomes de carbone dans la fraction aryle et 1 ou 2 atomes de carbone dans la fraction alkyle, ce groupe aralkyle étant éventuellement substitué par un atome d'halogène, un groupe alkyle contenant 1 à 4 atomes de carbone, un groupe halogénalkyle contenant 1 ou 2 atomes de carbone et 2 à 5 atomes d'halogènes, un groupe alcoxy et un groupe alkylthio contenant chacun 1 ou 2 atomes de carbone, un groupe cyano et par un groupe nitro,

ainsi que leurs sels d'addition d'acide physiologiquement compatibles.

2. Procédé de préparation d'aminopropiophénone-oximes de formule générale I suivant la revendication 1, caractérisé en ce que

a) on fait réagir des aminopropiophénones de formule générale II

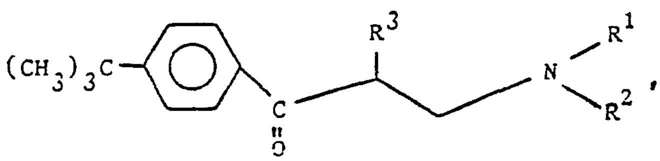

(II)

dans laquelle

$R^1$, $R^2$ et $R^3$ ont les significations indiquées dans la revendication 1,

avec des sels (de dérivés) d'hydroxylamine de formule générale III

$$H_2N—O—R^4 \qquad (III)$$

dans laquelle

$R^4$ a la signification indiquée ci-dessus,

en présence d'un diluant et d'un agent fixateur d'acide, et/ou

b) on fait éventuellement réagir des sels alcalins de dérivés d'oximes de formule générale IV que l'on obtient suivant le procédé a) et qui répondent à la formule générale IV

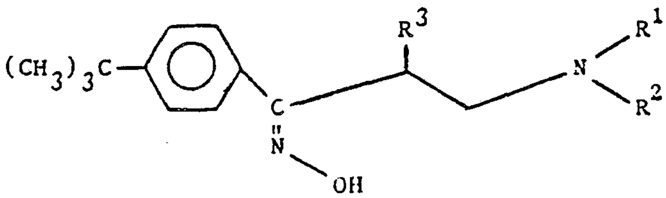

(IV)

dans laquelle

$R^1$, $R^2$ et $R^3$ ont les significations indiquées dans la revendication 1,

avec des halogénures de formule générale V

$$Z—R^5 \qquad (V)$$

dans laquelle

$R^5$ représente un groupe alkyle contenant 1 à 4 atomes de carbone, un groupe alcényle et un groupe alcinyle contenant chacun 2 à 4 atomes de carbone ou un groupe aralkyle contenant 6 à 10 atomes de carbone dans la fraction aryle et 1 ou 2 atomes de carbone dans la fraction alkyle, ce groupe aralkyle étant éventuellement substitué par un atome d'halogène, un groupe alkyle contenant 1 à 4 atomes de carbone, un groupe halogénalkyle contenant 1 ou 2 atomes de carbone et 2 à 5 atomes d'halogènes, un groupe alcoxy et un groupe alkylthio contenant chacun 1 ou 2 atomes de carbone, un groupe cyano et par un groupe nitro, et

Z représente un atome de chlore ou de brome,

en présence d'un diluant organique ou d'un système organique/inorganique à deux phases, ainsi qu'en présence d'un catalyseur de transfert de phases et, sur les composés de formule générale I ainsi obtenus, on fixe encore éventuellement un acide physiologiquement compatible.

3. Agents fongicides, caractérisés en ce qu'ils contiennent au moins une aminopropiophénone-oxime de formule générale I.

4. Procédé pour combattre les champignons sur les matières végétales et techniques, caractérisé en ce qu'on fait agir des aminopropiophénone-oximes de formule générale I sur les champignons ou leurs biotopes.

5. Utilisation d'aminopropiophénone-oximes de formule générale I pour combattre les champignons sur les matières végétales et techniques.

6. Procédé de préparation d'agents fongicides, caractérisé en ce qu'on mélange des aminopropiophénone-oximes de formule générale I avec des diluants et/ou des agents tensio-actifs.